(19) Europäisches Patentamt
European Patent Office
Office européen des brevets

(11) Publication number: **0 346 770 B1**

(12) # EUROPEAN PATENT SPECIFICATION

(45) Date of publication of patent specification: **11.01.95**

(51) Int. Cl.[6]: **C07D 207/452**, C07D 209/48, C07D 209/76, C08G 73/12

(21) Application number: **89110479.6**

(22) Date of filing: **09.06.89**

(54) **Curable fluorine containing polyimide.**

(30) Priority: **11.06.88 JP 144388/88**

(43) Date of publication of application: **20.12.89 Bulletin 89/51**

(45) Publication of the grant of the patent: **11.01.95 Bulletin 95/02**

(84) Designated Contracting States: **DE FR GB**

(56) References cited:
**EP-A- 0 032 745**

**CHEMICAL ABSTRACTS, vol. 106, no. 10, 9th March 1987, page 31, column 1,abstract no. 68089y, Columbus, Ohio, US; D.J. CAPO et al.: "An acetylenic-terminated fluorinated polyimide, properties and applications"**

**CHEMICAL ABSTRACTS, vol. 102, no. 6, 11th February 1985, page 92, column 1, abstract no. 47500c, Columbus, Ohio, US ; & JP-A-59176321**

(73) Proprietor: **DAIKIN INDUSTRIES, LIMITED**
**Umeda Center Building,**
**4-12, Nakazaki-nishi 2-chome,**
**Kita-ku**
**Osaka-shi, Osaka-fu (JP)**

(72) Inventor: **Kubo, Motonobu**
**4-13-1, Kumano-cho**
**Toyonaka-shi**
**Osaka-fu (JP)**
Inventor: **Kobayashi, Tsutomu**
**2-28-10, Torikai Shin-machi**
**Settsu-shi**
**Osaka-fu (JP)**

(74) Representative: **Hansen, Bernd, Dr.rer.nat. et al**
**Hoffmann, Eitle & Partner**
**Patentanwälte**
**Postfach 81 04 20**
**D-81904 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

### BACKGROUND OF THE INVENTION

#### Field of the Invention

The present invention relates to a novel curable fluorine-containing polyimide which is useful for preparing a cured resin showing high heat- and moisture-resistance and having a low refractive index and a low dielectric constant.

#### Description of the Related Art

The following compound, for example, has been known as a curable fluorine-containing polyimide having terminal unsaturated groups:

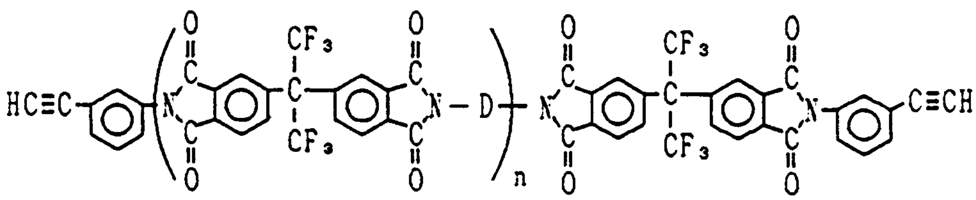

wherein D is a group derived from a diamine by the removal of two amino groups, and n is 1.0 on an average (cf. 18th International SAMPE Technical Conference, October 7 to 9, 1986, pages 710 to 721).

A cured resin prepared from such a fluorine-containing polyimide may be unsatisfactorily applied on electronic or optical parts due to its inadequate moisture resistance, a high refractive index and a high dielectric constant.

### SUMMARY OF THE INVENTION

An object of the present invention is to provide a curable fluorine-containing polyimide which is useful for preparing a cured resin showing high moisture resistance and having a low refractive index and a low dielectric constant.

The present invention provides a curable fluorine-containing polyimide of the formula:

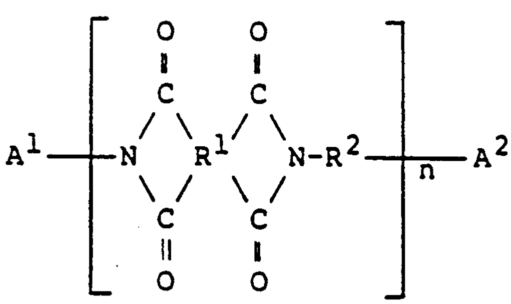

(I)

wherein $R^1$ is a group derived from an aromatic tetracarboxylic acid dianhydride by the removal of two acid anhydride groups,
$R^2$ is a group derived from an aromatic diamine by the removal of two amino groups,
$A^1$ is a residue of the formula:

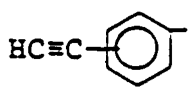 , 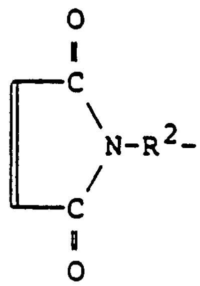 or 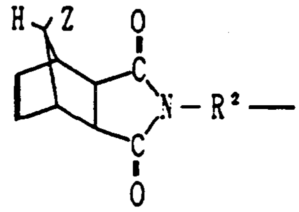

[wherein $R^2$ is the same as defined above, and Z is a hydrogen atom or an alkyl group having 1 to 5 carbon atoms],
$A^2$ is a residue of the formula:

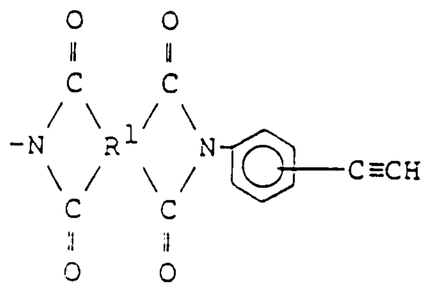 , 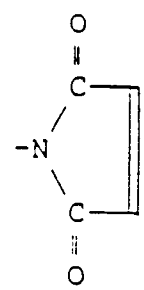 or 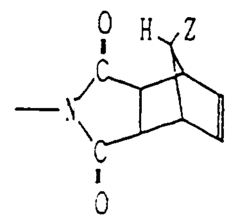

[wherein $R^1$ and Z are the same as defined above], and
n is a number of 0 to 90, and
at least one of the groups $R^1$ and $R^2$ in the formula (I) contains a substituted methylene group of the formula:

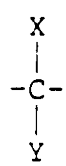

[wherein X is a residue of the formula:

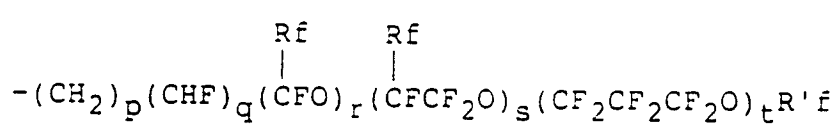

wherein Rf is a perfluoroalkyl group having 1 to 10 carbon atoms, R'F is a perfluoroalkyl group having 1 to 12 carbon atoms, p is an integer of 1 to 3, g is an integer of 0 to 3,
r is 0 or 1, s is an integer of 0 to 5 and t is an integer of 0 to 5, and
Y is the same as X or a hydrogen atom, an alkyl group having 1 to 8 carbon atoms or a fluoroalkyl group having 1 to 8 carbon atoms].

## DETAILED DESCRIPTION OF THE INVENTION

The present fluorine-containing polyimide includes the compounds of the following formulae:

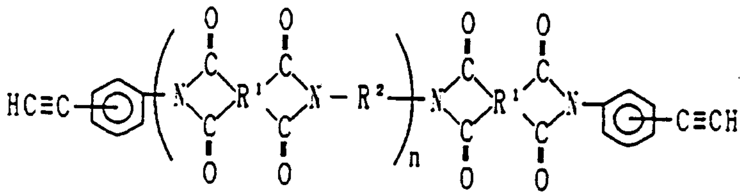

(I-1)

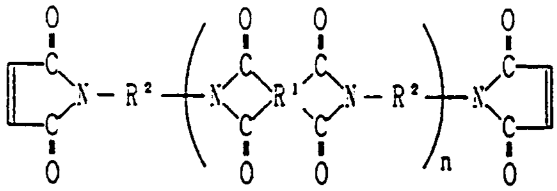

(I-2)

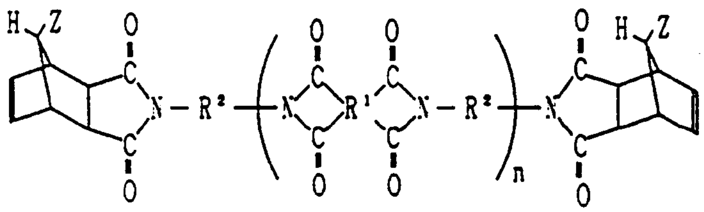

(I-3)

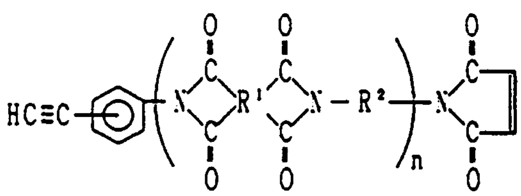

(I-4)

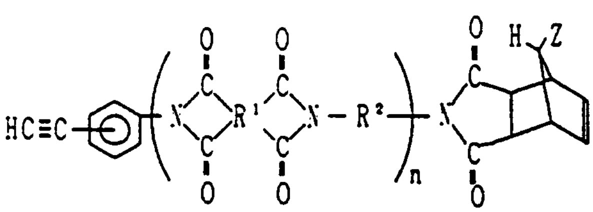

(I-5)

and

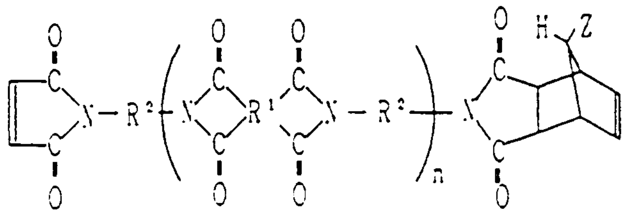

(I-6)

wherein $R^1$, $R^2$, Z and n are the same as defined above.

Examples of the polyimide of the present invention are shown as follows:

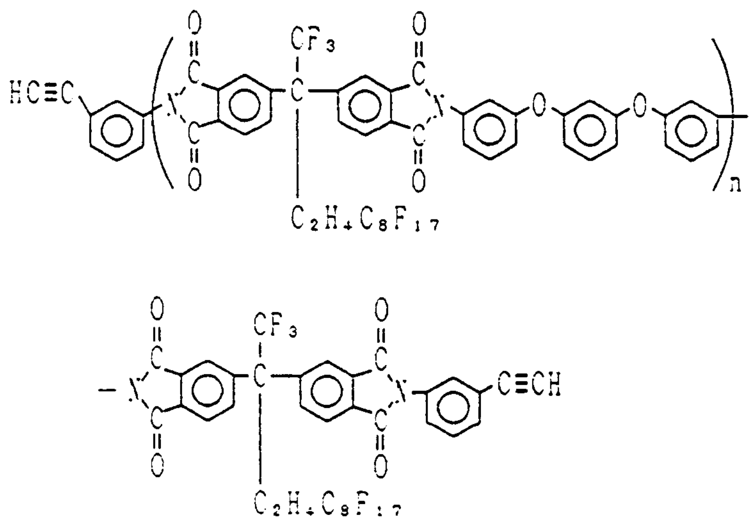

(1)

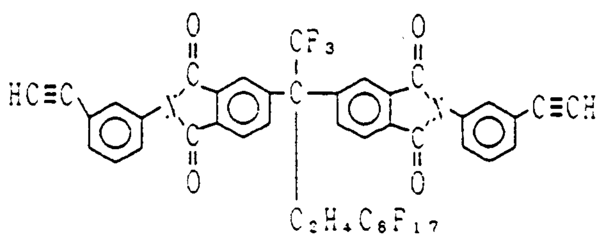

(2)

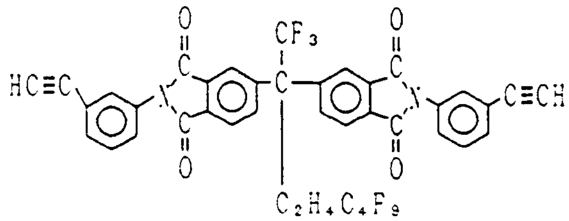

(3)

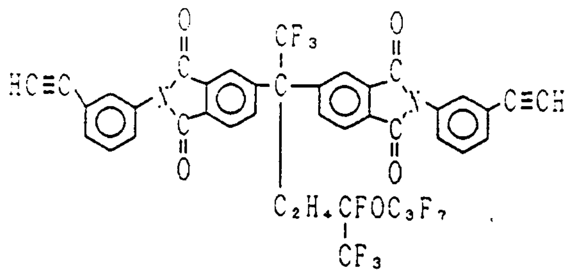

(4)

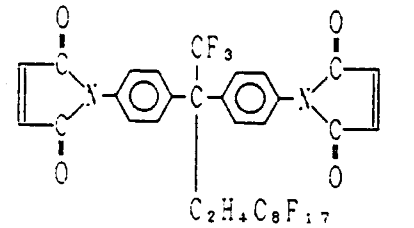

(5)

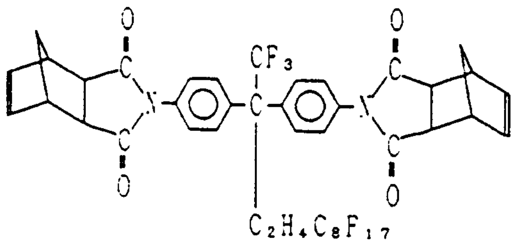

(6)

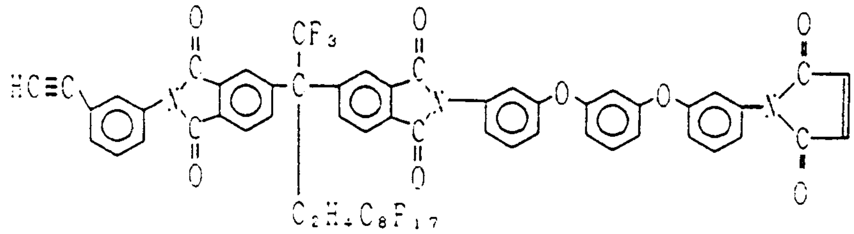

(7)

and

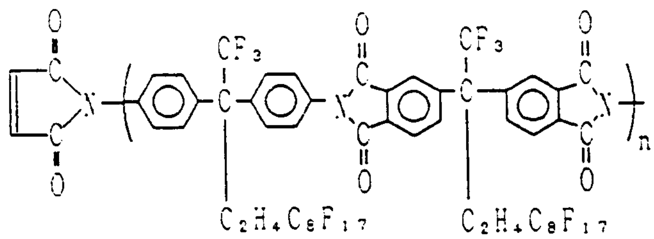

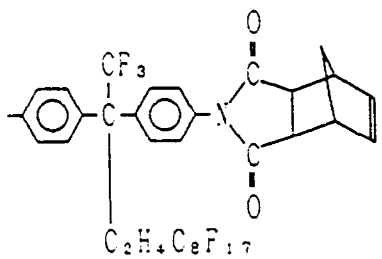

(8)

wherein n is the same as defined above.

The present fluorine-containing polyimide has a substituted methylene group of the following formula between aromatic rings in the repeating unit:

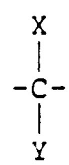

wherein X and Y are the same as defined above.

When n is 0 in the formula (I), the substituted methylene group should be contained in $R^1$ in the formula (I-1) and in $R^2$ in the formulae (I-2) and (I-3).

When n is not 0, at least one of the groups $R^1$ and $R^2$ in the main chain in the formula (I) should contain the substituted methylene group.

The groups $R^1$ and $R^2$ each containing the substituted methylene group can be represented as follows, respectively:

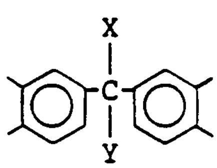

and

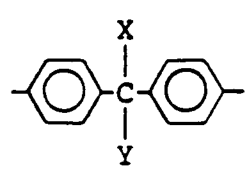

wherein X and Y are the same as defined above.

The present compound may have the following groups, for example, as $R^1$:

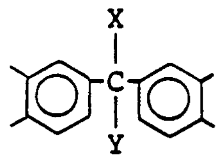

[wherein X and Y are the same as defined above],

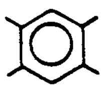 , 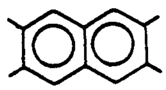 , 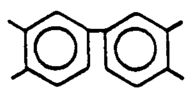 ,

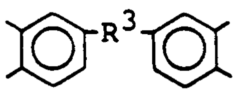

[wherein $R^3$ is -O-, -CO-,

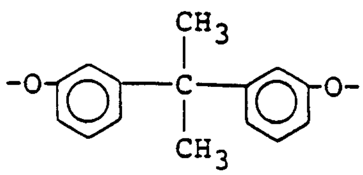 , 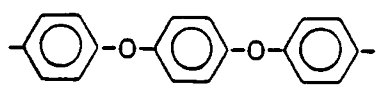 , $-SO_2-$, 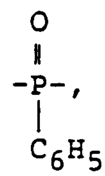 ,

$-C(CH_3)_2-$, $-C(CF_3)_2-$ or $-Si(CH_3)_2-$],

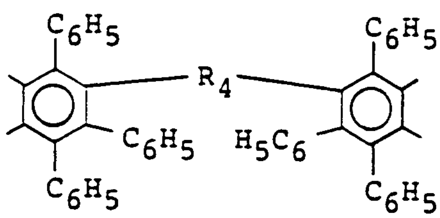

[wherein $R^4$ is $-C_6H_4-$, $-C_6H_4-O-C_6H_4-$ or $-C_6H_4-O-C_6H_4-O-C_6H_4-$], and

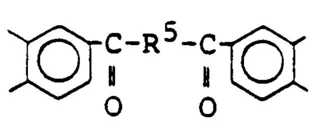

[wherein $R^5$ is -O-, $-O-(CH_2)_4-O-$, $-O-(CH_2)_6-O-$,

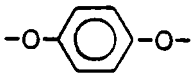, 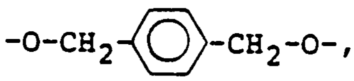, 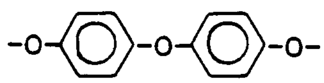,

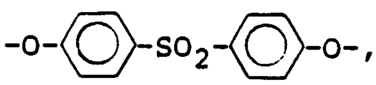, 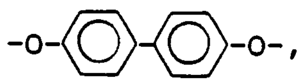, 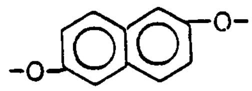,

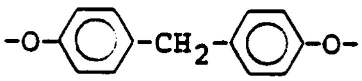 or 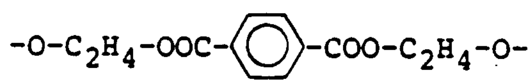].

The following groups, for example, may be present as $R^2$:

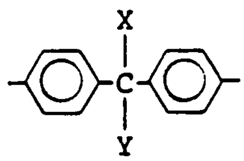

[wherein X and Y are the same as defined above],

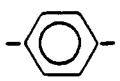, 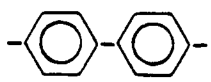, 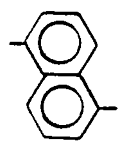,

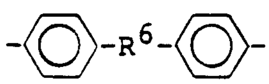

[wherein $R^6$ is -O-, -CO-, -S-, $-CH_2-$, $-C(CH_3)_2-$, $-C(CF_3)_2-$,$-SO_2-$,

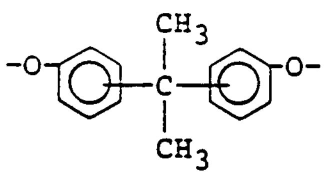, 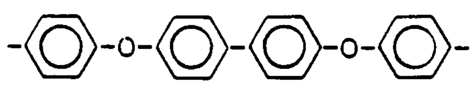

or $-Si(CH_3)_2-$, and

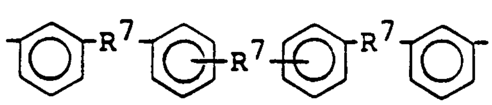

[wherein $R^7$ is -O-, $-SO_2-$, $-CH_2-$, -CO-, $-C(CH_3)_2-$ or -S-].

The present compound (I-1) can be prepared according to the following reaction scheme:

When n is not 0:

(n + 1) · 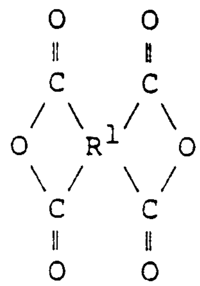 + n · $H_2N-R^2-NH_2$

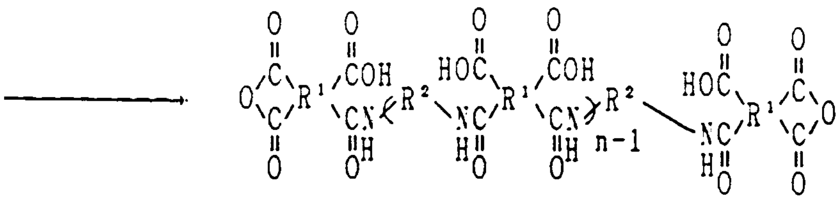 (II)

Compound (II) + 2 · 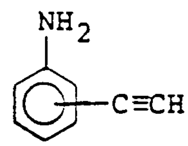 (ethynylaniline)

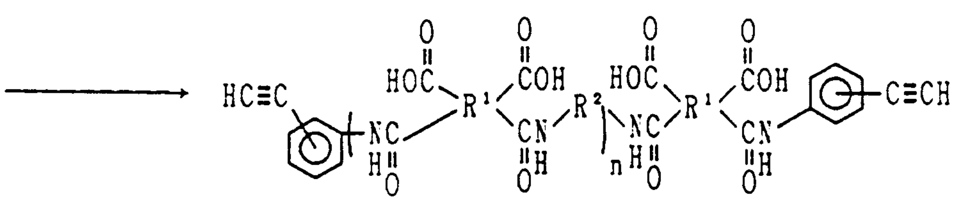 (III)

cyclodehydration → compound (I-1)

When n is 0:

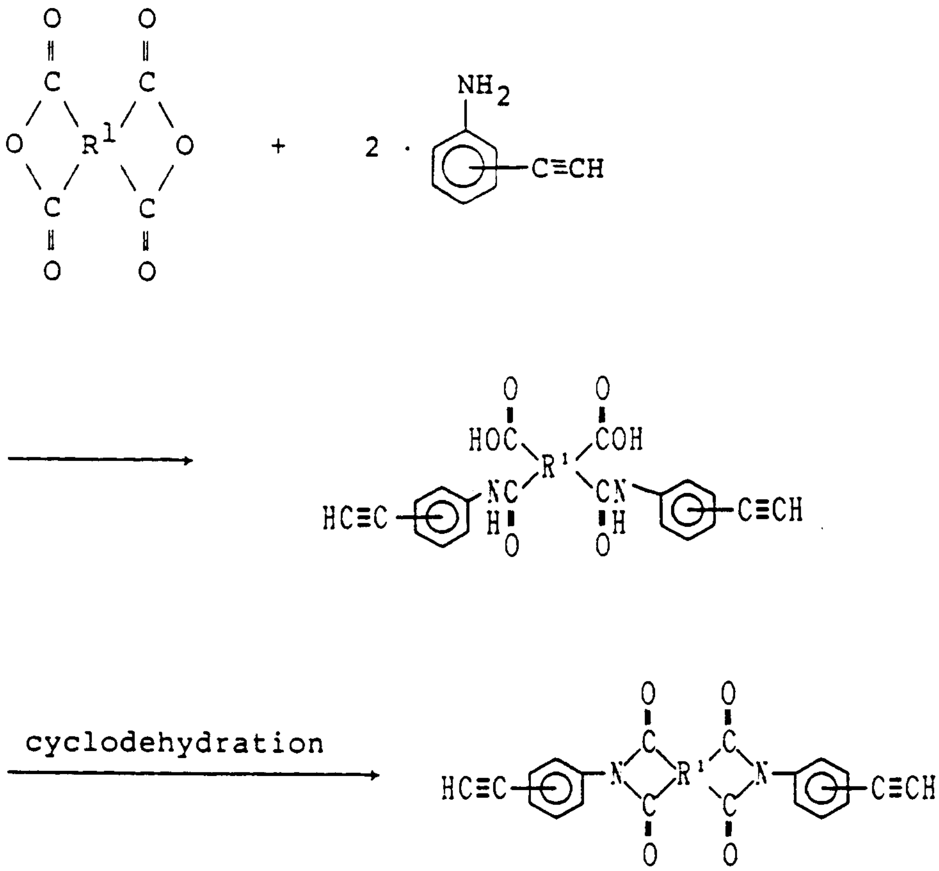


(IV)

(I-1)

The present fluorine-containing polyimide compounds (I-2) and (I-3) can be prepared according to the following reaction scheme.

When n is not 0:

(n + 1) · $H_2N-R^2-NH_2$ + n ·

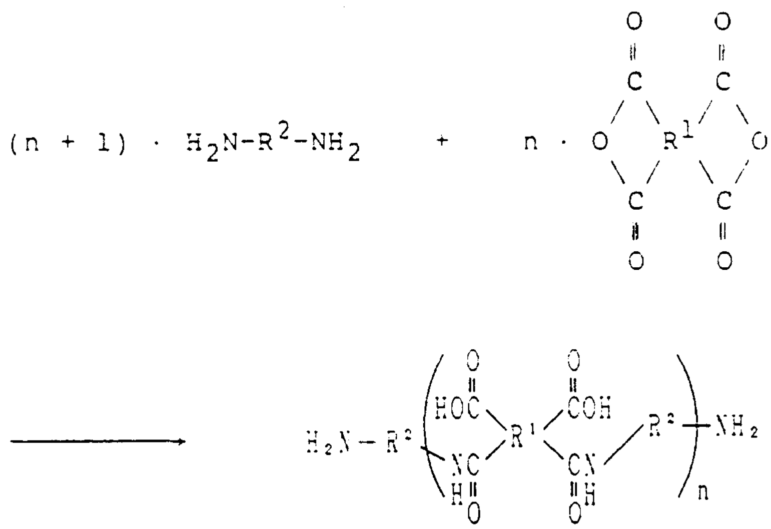

(VI)

Compound (VI) +

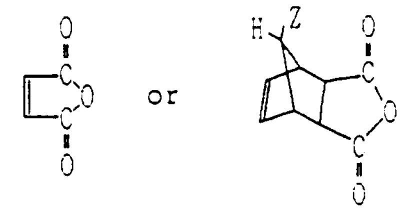

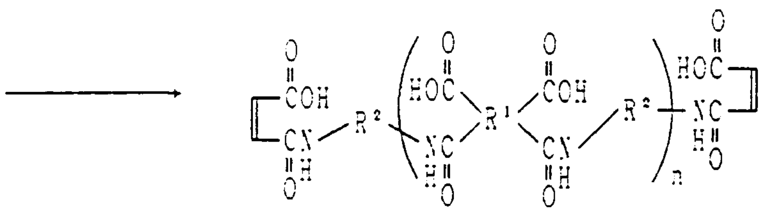

or

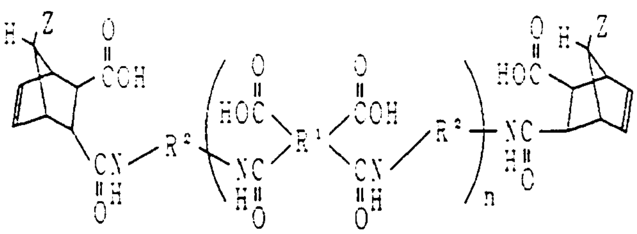

(VII)

cyclodehydration

Compound (I-2) or (I-3)

When n is 0:

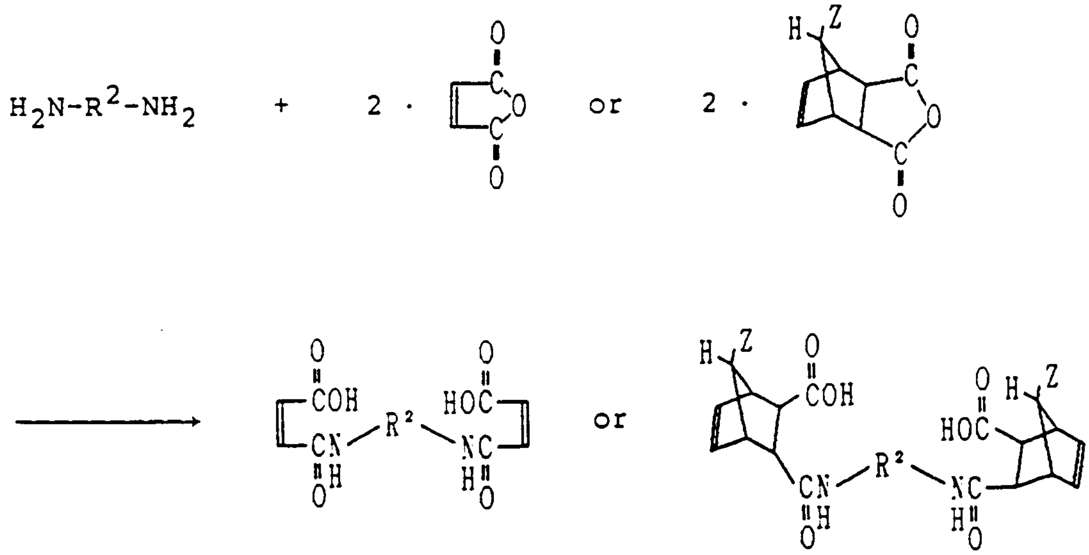

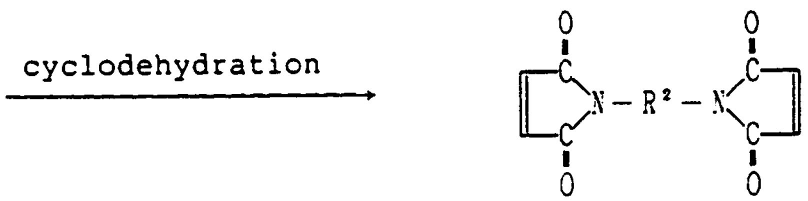


(I-2) or

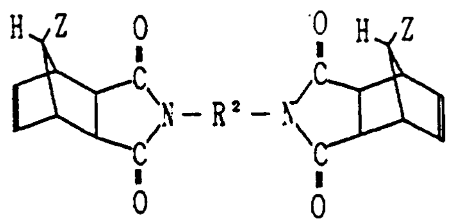

(I-3)

The present compounds (I-4) and (I-5), wherein n is 0, can be prepared as follows:

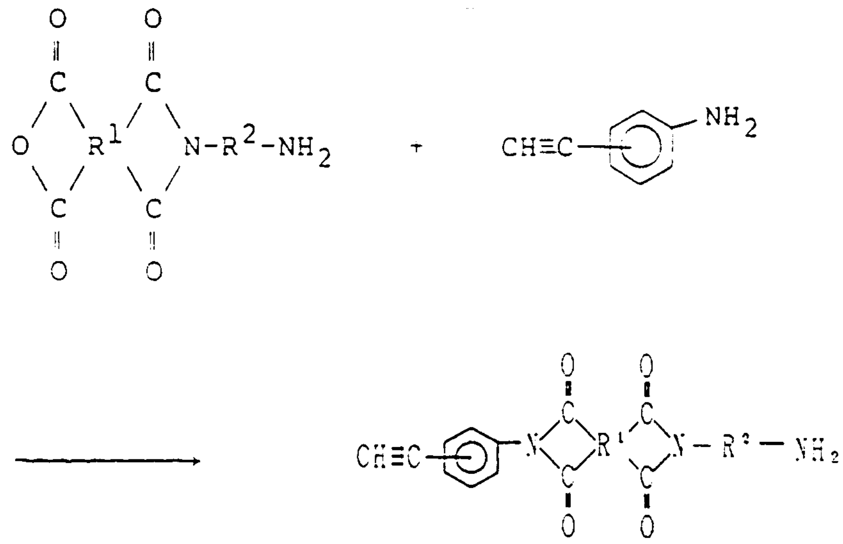

(VIII)

Compound (VIII) + 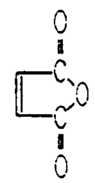 or 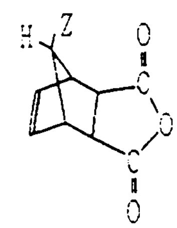

Compound (I-4) or (I-5)

The present fluorine-containing polyimide (I-6) can be obtained by the same way as for the production of the compound (I-2) or (I-3) but that a mixture of

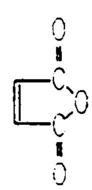 and 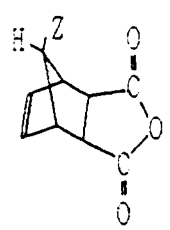

is used.

As shown in the reaction schemes, an amide acid is prepared as an intermediate. The reaction for the preparation of the amide acid is preferably carried out for 0.1 to 5 hours at a temperature in the range of from 0 to 15 °C, in particular 20 to 100 °C in a solvent such as tetrahydrofuran, acetone, dioxane, N-methyl-2-pyrrolidone, N,N-dimethylformamide, N,N-dimethylacetamide, dimethyl sulfoxide, diglyme, acetonitrile, 1,3-dimethyl-2-imidazolidinone or m-xylene hexafluoride with or without benzene, toluene or xylene.

Then, the amide acid is cyclodehydrated to form the present fluorine-containing polyimide. In this reaction, a dehydrating agent such as acetic anhydride, phosphorous pentoxide or conc. sulfuric acid is used. The reaction is preferably carried out in the presence of a catalyst and a base in a solvent. An oxide of an alkaline earth metal, or a carbonate, acetate, phosphate or sulfate of Fe(II) or (III), Ni(II), Mn(II) or (III), Cu(I) or (II) or Co(II) or (III) can be used as the catalyst. The base is, for example, trimethylamine, triethylamine, tributylamine or sodium acetate. The solvent previously used in the preparation of the amide

acid is preferably used as such in this reaction. The dehydrating agent is usually used in a 1 to 20 times molar amount per mol of the amide acid. The catalyst and the base are added, based on the amount of the amide acid, in an amount in the range of from 1 x $10^{-2}$ to 1 x 10 mol % and in an amount in the range of from 5 to 150 mol %, respectively. It is preferred to carry out the reaction for 2 to 24 hours at a temperature in the range of from 100 to 250 °C, in particular from 120 to 280 °C.

The present fluorine-containing polyimide compounds (I-1), (I-2) and (I-3) can be cured by heating at a temperature in the range of from 200 to 500 °C, preferably from 250 to 400 °C for 0.1 to 5 hours. Prior to curing, a compound containing an ethynyl or ethylene group, such as an acrylic, styrene, vinylether or vinylester compound may be added to the present compound. To the present compounds (I-2) and (I-3), a diamine compound can also be added.

In addition, a filler may be added to the fluorine-containing polyimide. The filler used is, for example, calcium carbonate, silica, alumina, titania, aluminium hydroxide, aluminium silicate, zirconium silicate, zircon, glass, talc, mica, graphite, aluminium, copper or iron in a powder or short fibrous form.

The present fluorine-containing polyimide can be molded by, for example, compression or transfer molding. The molding may be easily carried out by the addition of the filler. It is also possible to coat the present compound as a varnish on a substrate.

The present fluorine-containing polyimide can be suitably used as a heat- and moisture-resistant material in, for example, a matrix resin for an aerospace structural material; a base resin for a heat resistant adhesive and a conductive adhesive; a rigid or flexible printed board material; a sealing agent for electric parts such as a semiconductor device; a passivation film; an interlayer insulating film; an α-ray blocking film; a protecting film on a surface against an external force; an insulating and reinforcing material for wire bonding parts; a masking material for ion implantation and lift-off; an oriented film for a liquid crystal display device; a sealing and protecting coating agent for optical parts such as a glass fiber and LED; and a protecting film for a solar battery.

## PREFERRED EMBODIMENTS OF THE INVENTION

The invention is further illustrated by the following Examples.

### Example 1

To a stirred mixture of the compound (32.89 g; 0.04 mol) of the formula:

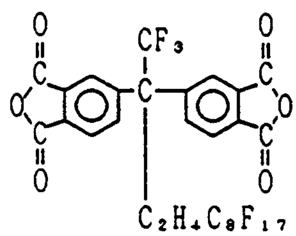

with N-methylpyrrolidone (NMP; 60 ml) in a 200 ml three necked flask, a solution of 1,3-bis(3-aminophenoxy)benzene (APB; 5.85 g; 0.02 mol) in NMP (15 ml) was slowly dropwise added at a room temperature. After stirring for one hour at 50 °C, 3-aminophenylacetylene (APA; 5.04 g; 0.043 mol) in NMP (6 ml) was added and heated. Then, benzene was added and heated under reflux for 20 hours at 140 °C with removing water by a Dean-Stark condenser. The reaction solution was cooled to the room temperature and poured into ethyl alcohol to precipitate a reaction product. The reaction product was filtered and washed three times with ethyl alcohol to remove NMP. Then, ethyl alcohol was distilled off under a reduced pressure. After drying for 24 hours at 80 °C under a reduced pressure, the compound of the formula:

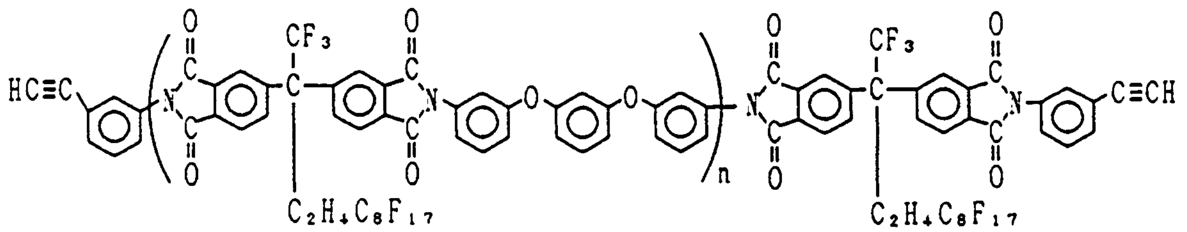

wherein n is 1.0 on an average,

was obtained. Yield: 32.43 g (0.015 mol; 77 %). The value n was calculated by a quantitative analysis of terminal acetylene according to L. Barnes, Jr., Anal. Chem., 31 (3), 405 (1959).

$^1$H-NMR ($CDCl_3$/TMS) $\delta$ [ppm]: 8.1 - 6.7 (m, 32H), 3.11 (s, 2H), 3.0 - 1.6 (broad, 8H).

$^{19}$F-NMR $CDCl_3$/TFA) $\delta$ [ppm]: - 13.1 (s, 6F), 2.1 (t, 6F), 35.7 (broad, 4F), 43.0 (broad, 12F), 44.1 (broad, 8F), 47.3 (broad, 4F).

IR, $\nu$ [$cm^{-1}$]: 3300, 1780, 1725, 1585, 1480, 1430, 1375, 1240, 1200, 1170, 1140, 1100, 1010, 980, 910, 870, 840, 790, 740, 720, 710.

Example 2

In a 200 ml three necked flask, a mixture of the compound (41.12 g; 0.05 mol) of the formula:

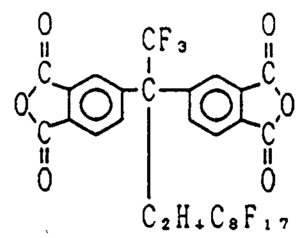

with APA (6.44 g; 0.055 mol) and NMP (70 ml) was stirred for one hour at 50 °C and then further heated. Then, to the mixture, toluene was added and heated under reflux for 15 hours at 140 °C with removing water by a Dean-Stark condenser. The reaction solution was cooled to a room temperature and poured into ethyl alcohol to precipitate a reaction product. The reaction product was filtered and washed three times with ethyl alcohol to remove NMP. Then, ethyl alcohol was distilled off under a reduced pressure. After drying for 24 hours at 80 °C under a reduced pressure, the compound of the formula:

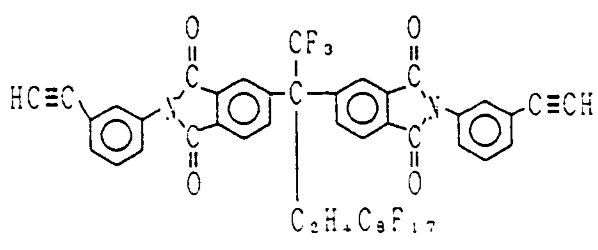

was obtained. Yield: 34.30 g (0.034 mol; 67 %).

$^1$H-NMR ($CDCl_3$/TMS) $\delta$ [ppm]: 8.1 - 7.2 (m, 14H), 3.11 (s, 2H), 3.0 - 1.6 (broad, 4H).

$^{19}$F-NMR ($CDCl_3$/TFA) $\delta$ [ppm]: - 13.1 (s, 3F), 2.1 (t, 3F), 35.8 (broad, 2F), 43.1 (broad, 6F), 44.0 (broad, 4F), 47.3 (broad, 2F).

IR, $\nu$ [$cm^{-1}$]: 3300, 1780, 1720, 1600, 1580, 1480, 1430, 1370, 1230, 1200, 1170, 1140, 1100, 1005, 970, 785, 740, 710, 700.

Example 3

In a 200 ml three necked flask, a mixture of the compound (43.56 g; 0.07 mol) of the formula:

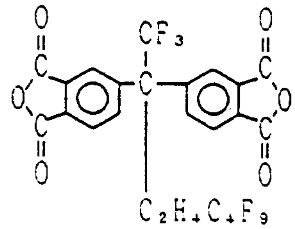

with APA (8.79 g; 0.075 mol) and NMP (80 ml) was stirred for one hour at 50 °C and then further heated. Then, to the mixture, toluene was added and heated under reflux for 20 hours at 140 °C with removing water by a Dean-Stark condenser. The reaction solution was cooled to a room temperature and poured into ethyl alcohol to precipitate a reaction product. The reaction product was filtered and washed three times with ethyl alcohol to remove NMP. Then, ethyl alcohol was distilled off under a reduced pressure. After drying for 24 hours at 80 °C under a reduced pressure, the compound of the formula:

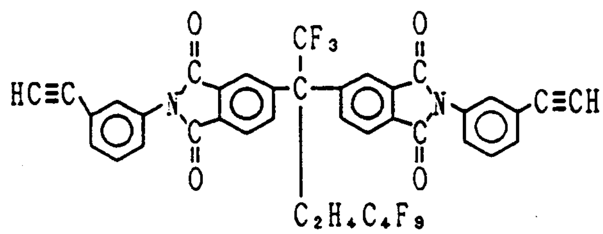

was obtained. Yield: 41.33 g (0.050 mol; 72 %).

$^1$H-NMR ($CDCl_3$/TMS) $\delta$ [ppm]: 8.1 - 7.2 (m, 14H), 3.12 (s, 2H), 3.0 - 1.6 (broad, 4H).

$^{19}$F-NMR ($CDCl_3$/TFA) $\delta$ [ppm]: - 13.3 (s, 3F), 2.3 (t, 3F), 35.2 (broad, 2F), 45.2 (broad, 2F), 47.3 (broad, 2F).

IR, $\nu$ [$cm^{-1}$] : 3300, 1780, 1720, 1600, 1570, 1540, 1480, 1430, 1370, 1235, 1200, 1170, 1140, 1100, 1010, 970, 870, 790, 740, 710, 700.

Example 4

In a 200 ml three necked flask, a mixture of the compound (41.30 g; 0.06 mol) of the formula:

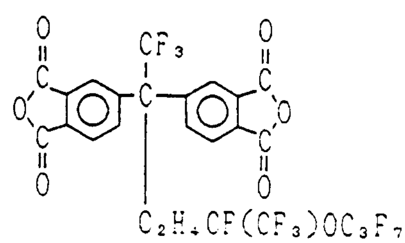

with APA (7.73 g; 0.066 mol) and NMP (70 ml) was stirred for one hour at 50 °C and then further heated. Then, to the mixture, toluene was added and heated under reflux for 15 hours at 140 °C with removing water by a Dean-Stark condenser. The reaction solution was cooled to a room temperature and poured into ethyl alcohol to precipitate a reaction product. The reaction product was filtered and washed three times with ethyl alcohol to remove NMP. Then, ethyl alcohol was distilled off under a reduced pressure. After drying for 24 hours at 80 °C under a reduced pressure, the compound of the formula:

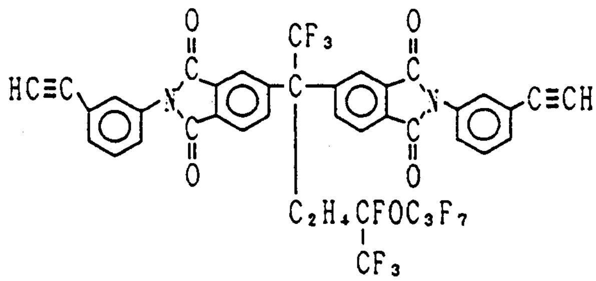

was obtained. Yield: 39.69 g (0.045 mol; 75 %).

$^1$H-NMR $CDCl_3$/TMS) $\delta$ [ppm]: 8.1 - 7.1 (m, 14H), 3.11 (s, 2H), 3.0 - 1.6 (broad, 4H).

$^{19}$F-NMR ($CDCl_3$/TMS) $\delta$ [ppm]: - 12.2 (s, 3F), 2.9 (t, 3F), 3.1 (broad, 2F), 4.7 (d, 3F), 51.3 (broad, 1F), 52.8 (s, 2F).

IR, $\nu$ [$cm^{-1}$]: 3300, 1780, 1720, 1600, 1570, 1480, 1430, 1370, 1230, 1200, 1160, 1005, 970, 840, 750.

Example 5

To a stirred mixture of the compound (35.62 g; 0.05 mol) of the formula:

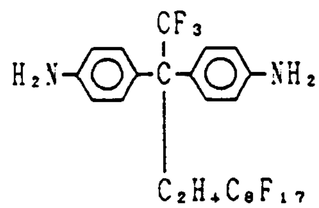

with m-xylene hexafluoride (m-XHF; 150 ml) In a 500 ml four necked frask, a solution of maleic anhydride (12.7 g; 0.13 mol) in m-XHF (150 ml) was slowly dropwise added at 50 °C and then stirred for 30 minutes at the same temperature. Then, benzyltrimethylammonium chloride (BTMAC; 0.5 g), $Ni(OAc)_2 \cdot 4H_2O$ (0.5 g) and conc. sulfuric acid (1.2 ml) were added to the reaction mixture and heated under reflux for 10 hours with removing water by a Dean-Stark condenser. An organic phase was neutralized with an aqueous $Na_2CO_3$ solution, washed two times with an aqueous NaCl solution and dried over anhydrous $Na_2SO_4$. Then, m-XHF was distilled off under a reduced pressure to obtain the compound of the formula:

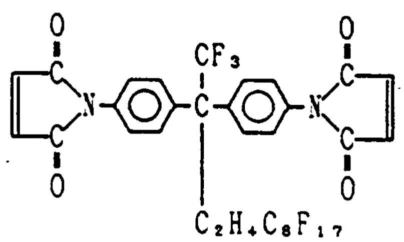

Yield: 28.10 g (0.032 mol; 64 %).

$^1$H-NMR ($CDCl_3$/TMS) $\delta$ [ppm]: 7.43 (s, 8H), 6.88 (s, 4H), 3.0 - 1.5 (broad, 4H).

$^{19}$F-NMR $CDCl_3$/TFA) $\delta$ [ppm]: - 12.8 (s, 3F), 2.2 (t, 3F), 35.9 (broad, 2F), 43.1 (broad, 6F), 43.7 (broad, 4F), 47.3 (broad, 2F).

IR, $\nu$ [$cm^{-1}$]: 3500, 1720, 1610, 1520, 1400, 1380, 1200, 1140, 1060, 1020, 1000, 970, 950, 830, 700.

Example 6

To a stirred mixture of the compound (35.62 g; 0.05 mol) of the formula:

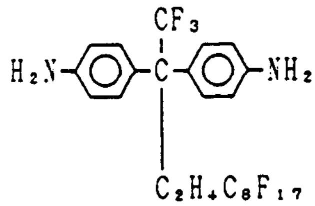

with m-XHF (150 ml) in a 500 ml four necked frask, a solution of 5-norbornene-2,3-dicarboxylic anhydride (21.3 g; 0.13 mol) in m-XHF was slowly dropwise added at 50 °C and then stirred for 30 minutes at the same temperature. Then, BTMAC (0.5 g), $Ni(OAc)_2 \cdot 4H_2O$ (0.5 g) and conc. sulfuric acid (1.2 ml) were added to the reaction mixture and heated under reflux for 10 hours with removing water by a Dean-Stark condenser. An organic phase was neutralized with an aqueous $Na_2CO_3$ solution, washed two times with an aqueous NaCl solution and dried over anhydrous $Na_2SO_4$. Then, m-XHF was distilled off under a reduced pressure to obtain the compound of the formula:

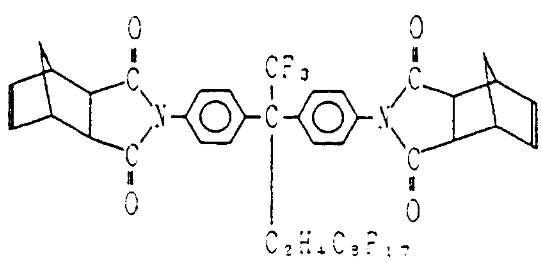

Yield: 34.66 g (0.035 mol; 69 %).

$^1$H-NMR ($CDCl_3$/TMS) $\delta$ [ppm]: 7.25 (q, 8H), 6.23 (s, 4H), 3.43 (m, 8H), 3.1 - 1.6 (broad, 4H), 1.7 (q, 4H).

$^{19}$F-NMR ($CDCl_3$/TMS) $\delta$ [ppm]: - 13.0 (s, 3F), 2.2 (t, 3F), 35.8 (broad, 2F), 43.1 (broad, 6F), 44.0 (broad, 4F), 47.4 (broad, 2F).

IR, $\nu$ [cm$^{-1}$]: 3400, 1710, 1510, 1375, 1200, 1170, 1140, 1000, 820, 720, 700.

Example 7

A 50 % by weight solution of the present fluorine-containing polyimide of Example 1 in NMP was casted on a stainless steel plate and heated for 20 minutes at 80 °C, 20 minutes at 150 °C and then one hour at 250 °C to form a cured resin.

The obtained resin was subjected to the following examinations.

As a comparison, a fluorine-containing polyimide of the formula:

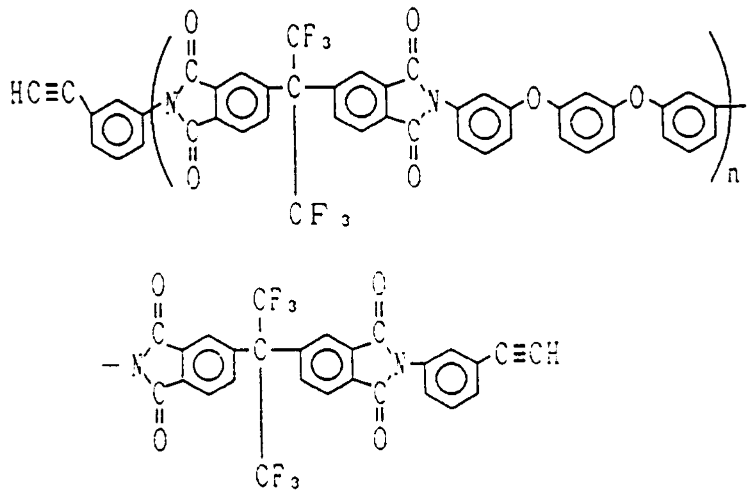

wherein n is 1.0 on an average,
was used.

(i) Water absorption

A specimen (76.2 mm x 25.4 mm x 50 $\mu$m) of the cured resin was dried for 24 hours at 50 ± 3 °C, cooled in a desiccator and then weighed ($W_1$). The specimen was immersed in distilled water at 23 ± 1 °C for 24 hours. Then, after wiping water off, the specimen was weighed ($W_2$). The water absorption (A) of the resin was calculated as follows:

$$A\ (\%) = \frac{W_2 - W_1}{W_1} \times 100$$

(ii) Refractive index

A refractive index of the cured resin was measured at 23 °C by using an Abbe refractometer.
The results are shown in the following Table 1.

Table 1

| Resin | Water absorption | Refractive index ($n_D^{23}$) |
|---|---|---|
| Example 1 | 0.25 | 1.468 |
| Comparison | 1.4 | 1.617 |

It was found that the refractive index of the present compound of Example 1 approximated to that of quartz ($n_D^{23}$ = 1.46).

## Claims

**1.** A curable fluorine-containing polyimide of the formula:

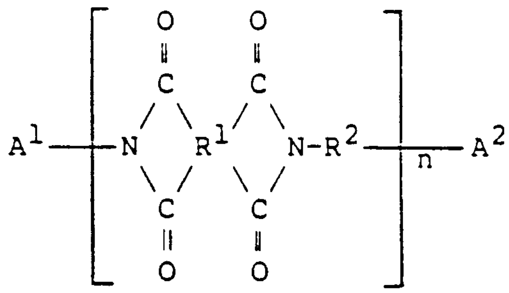 (I)

wherein $R^1$ is a group derived from an aromatic tetracarboxylic acid dianhydride by the removal of two acid anhydride groups,
$R^2$ is a group derived from an aromatic diamine by the removal of two amino groups,
$A^1$ is a residue of the formula:

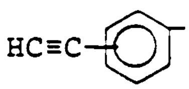 , 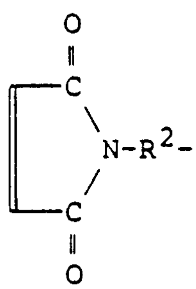 or 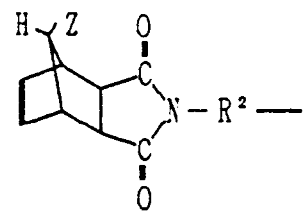

[wherein $R^2$ is the same as defined above, and Z is a hydrogen atom or an alkyl group having 1 to 5 carbon atoms],
$A^2$ is a residue of the formula:

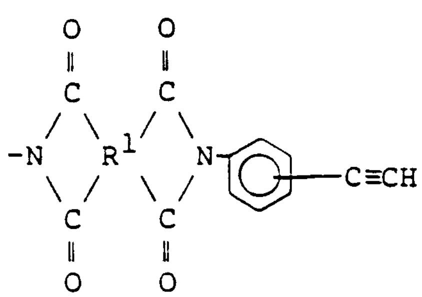 , 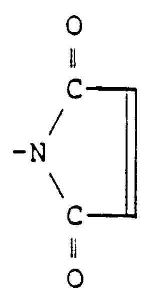 or 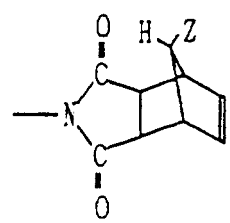

[wherein $R^1$ and Z are the same as defined above], and
n is a number of 0 to 90, and
at least one of the groups $R^1$ and $R^2$ in the formula (I) contains a substituted methylene group of the formula:

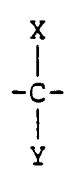

[wherein X is a residue of the formula:

$$-(CH_2)_p(CHF)_q(\overset{\overset{Rf}{|}}{C}FO)_r(\overset{\overset{Rf}{|}}{C}FCF_2O)_s(CF_2CF_2CF_2O)_tR'f$$

wherein Rf is a perfluoroalkyl group having 1 to 10 carbon atoms, R'F is a perfluoroalkyl group having 1 to 12 carbon atoms, p is an integer of 1 to 3, q is an integer of 0 to 3, r is 0 or 1, s is an integer of 0 to 5 and t is an integer of 0 to 5, and

Y is the same as X or a hydrogen atom, an alkyl group having 1 to 8 carbon atoms or a fluoroalkyl group having 1 to 8 carbon atoms].

**2.** The polyimide according to claim 1, which is a compound selected from the group consisting of the compounds of the formulae:

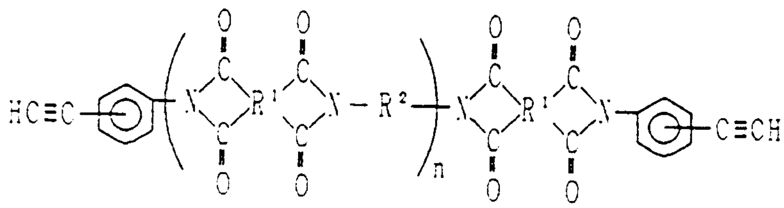

(I-1)

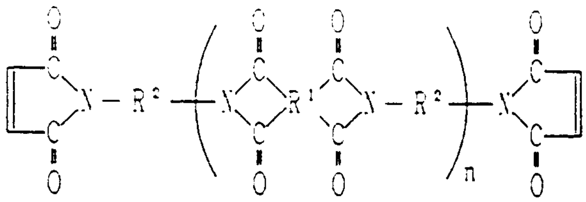

(I-2)

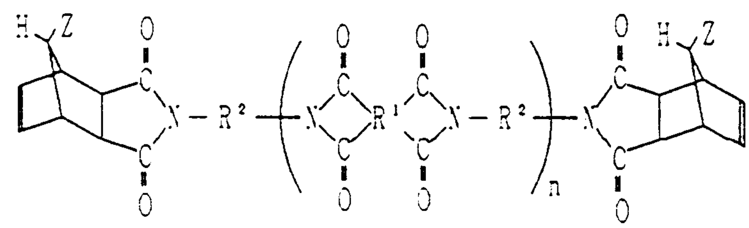

(I-3)

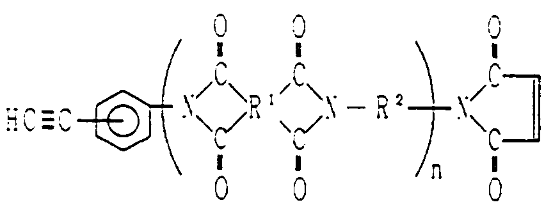

(I-4)

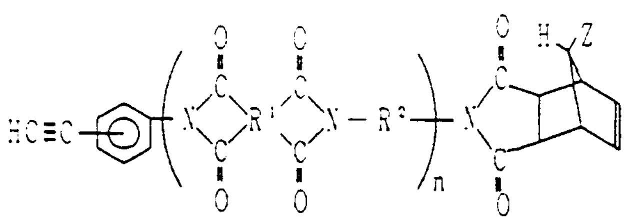

(I-5)

and

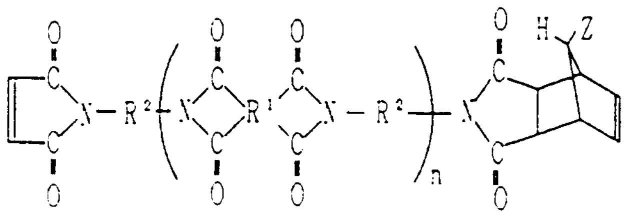

(I-6)

wherein $R^1$, $R^2$, Z and n are the same as defined above.

**3.** The polyimide according to claim 2, wherein, when n is 0 in the formula (I), the substituted methylene group should be contained in $R^1$ in the formula (I-1) and in $R^2$ in the formulae (I-2) and (I-3).

**4.** The polyimide according to claim 2, wherein, when n is not 0, at least one of the groups $R^1$ and $R^2$ in the main chain in the formula (I) should contain the substituted methylene group.

**Patentansprüche**

**1.** Härtbares fluorhaltiges Polyimid der Formel:

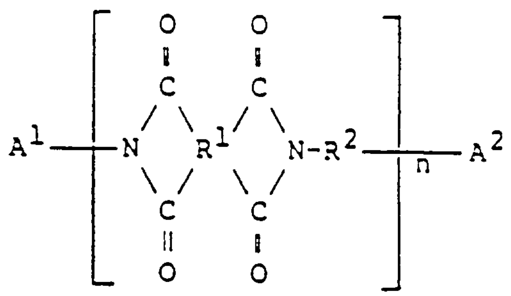

(I)

worin $R^1$ eine Gruppe ist, die sich von einem aromatischen Tetracarbonsäuredianhydrid durch die Entfernung von zwei Säureanhydridgruppen ableitet; worin $R^2$ eine Gruppe ist, die sich von einem aromatischen Diamin durch die Entfernung von zwei Aminogruppen ableitet, worin $A^1$ ein Rest der Formel ist:

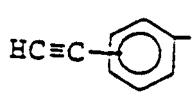

,

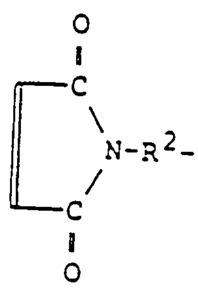

oder

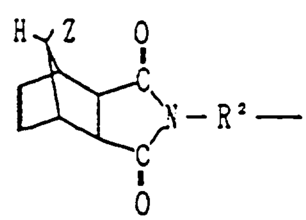

(worin $R^2$ gleich ist wie oben definiert, und worin Z ein Wasserstoffatom oder eine Alkylgruppe mit 1 bis 5 Kohlenstoffatomen ist), worin $A^2$ ein Rest der Formel ist:

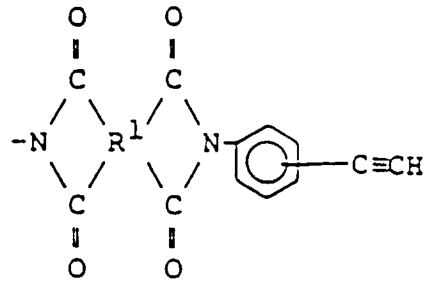 , 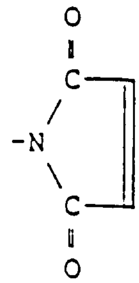 oder 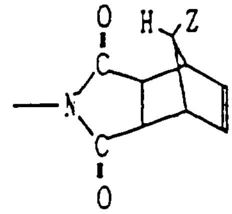

(worin $R^1$ und Z gleich sind wie oben definiert), und worin n eine Zahl von 0 bis 90 ist und wobei zumindest eine der Gruppen $R^1$ und $R^2$ in der Formel (I) eine substituierte Methylengruppe der Formel umfaßt

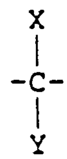

(worin X ein Rest der Formel ist:

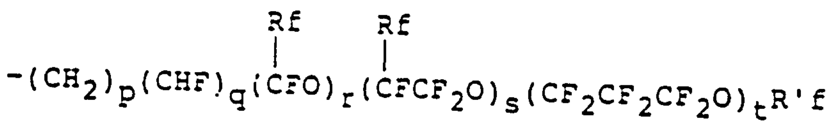

worin Rf eine Perfluorakylgruppe mit 1 bis 10 Kohlenstoffatomen ist, worin R'f eine Perfluoralkylgruppe mit 1 bis 12 Kohlenstoffatomen ist, worin p eine ganze Zahl von 1 bis 3, q eine ganze Zahl von 0 bis 3, r 0 oder 1, s eine ganze Zahl von 0 bis 5 und t eine ganze Zahl von 0 bis 5 ist, und worin Y gleich ist wie X oder ein Wasserstoffatom, eine Alkylgruppe mit 1 bis 8 Kohlenstoffatomen oder eine Fluoroalkylgruppe mit 1 bis 8 Kohlenstoffatomen ist).

**2.** Polyimid nach Anspruch 1, das eine Verbindung ist, ausgewählt aus der Gruppe, bestehend aus den Verbindungen mit den folgenden Formeln:

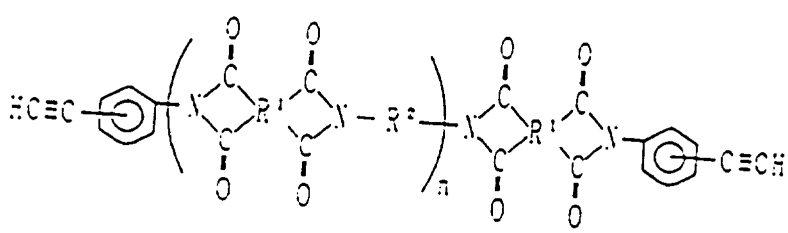

(I-1)

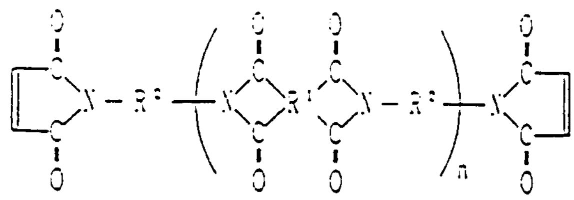

(I-2)

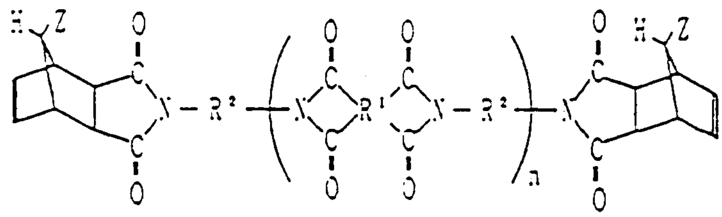

(I-3)

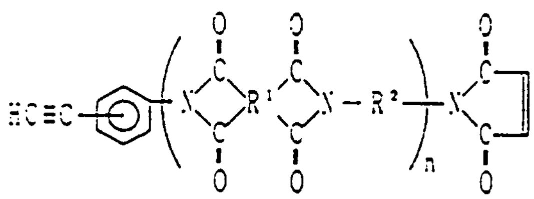

(I-4)

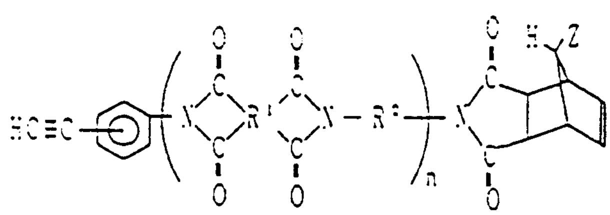

(I-5)

und

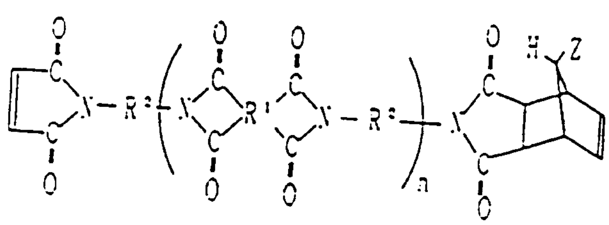

(I-6)

worin $R^1$, $R^2$, Z und n gleich sind wie oben definiert.

**3.** Polyimid nach Anspruch 2, worin die substituierte Methylengruppe in $R^1$ in der Formel (I-1) und in $R^2$ in den Formeln (I-2) und (I-3) enthalten ist, wenn n in der Formel (I) 0 ist.

**4.** Polyimid nach Anspruch 2, worin zumindest eine der Gruppen $R^1$ und $R^2$ in der Hauptkette in der Formel (I) die substituierte Methylengruppe enthält, wenn n nicht 0 ist.

## Revendications

**1.** Polyimide durcissable contenant du fluor de formule :

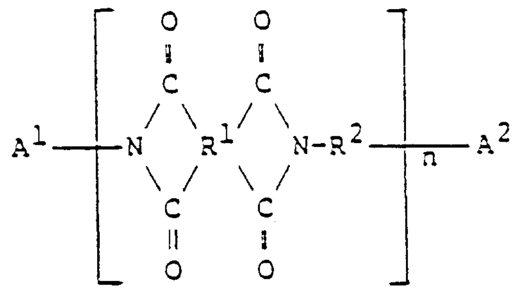

(I)

dans laquelle $R^1$ est un groupe dérivé d'un dianhydride d'acide aromatique tétracarboxylique par l'élimination de deux groupes anhydrides,
$R^2$ est un groupe dérivé d'une diamine aromatique par l'élimination de deux groupes amino,
$A^1$ est un reste de formule :

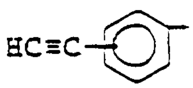 , 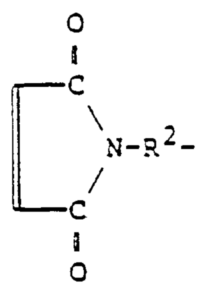 ou 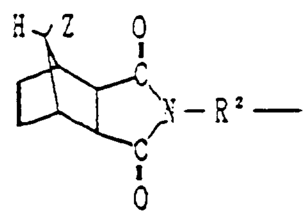

[dans lesquelles $R^2$ est tel que défini ci-dessus, et Z est un atome d'hydrogène ou un groupe alkyle ayant 1 à 5 atomes de carbone],
$A^2$ est un reste de formule :

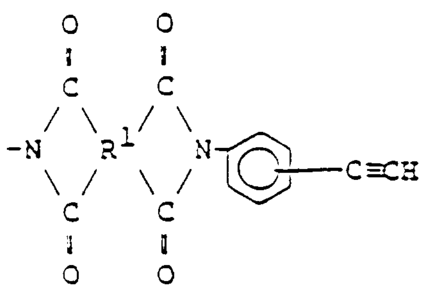 , 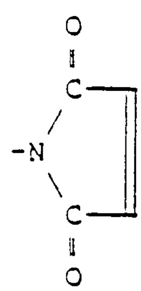 ou 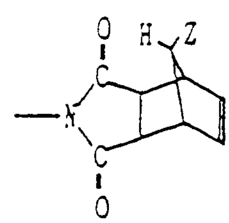

[dans lesquelles $R^1$ et Z sont tels que définis ci-dessus], et
n est un nombre de 0 à 90, et
au moins l'un des groupes $R^1$ et $R^2$ de formule (I) contient un groupe méthylène substitué de formule :

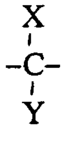

[dans laquelle X est un reste de formule :

$$-(CH_2)_p(CHF)_q(\overset{Rf}{\overset{|}{C}}FO)_r(\overset{Rf}{\overset{|}{C}}FCF_2O)_s(CF_2CF_2CF_2O)_tR'f$$

dans laquelle Rf est un groupe perfluoroalkyle ayant de 1 à 10 atomes de carbone, R'f est un groupe perfluoroalkyle ayant de 1 à 12 atomes de carbone, p est un entier de 1 à 3, q est un entier de 0 à 3, r est 0 ou 1, s est un entier de 0 à 5 et t est un entier de 0 à 5, et Y est identique à X ou est un atome d'hydrogène, un groupe alkyle ayant de 1 à 8 atomes de carbone ou un groupe fluoroalkyle ayant de 1 à 8 atomes de carbone].

**2.** Polyimide selon la revendication 1, qui est un composé choisi dans le groupe constitué des composés de formules :

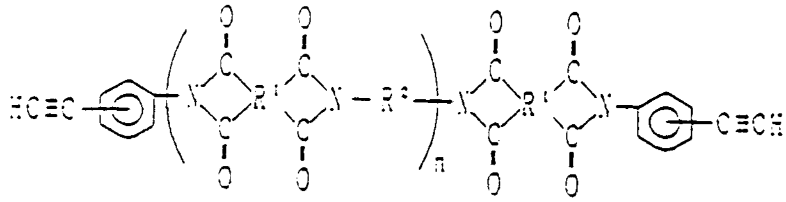

(I-1)

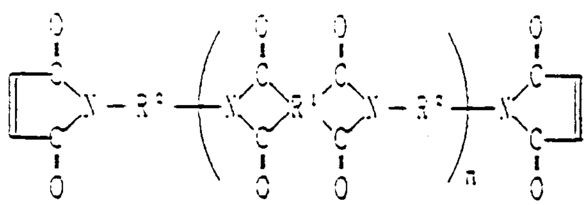

(I-2)

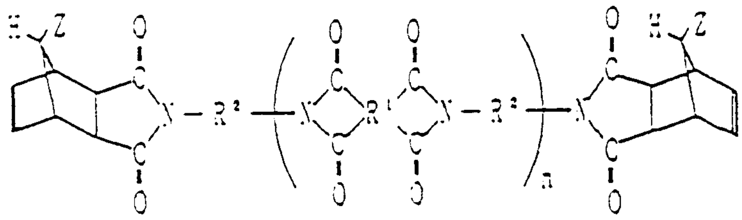

(I-3)

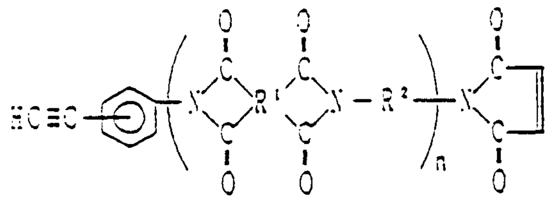

(I-4)

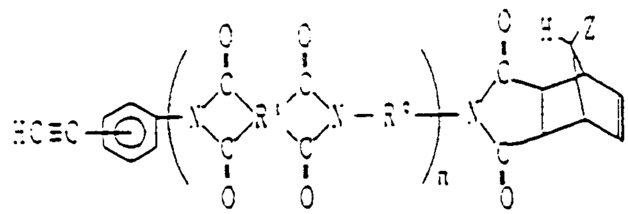

(I-5)

et

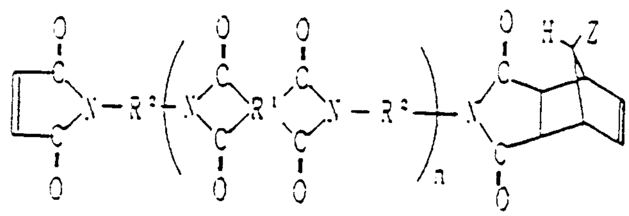

(I-6)

dans lesquelles $R^1$, $R^2$, Z et n sont tels que définis ci-dessus.

**3.** Polyimide selon la revendication 2, dans lequel, quand n est 0 dans la formule (I), le groupe méthylène substitué peut être compris dans $R^1$ dans la formule (I-1) et dans $R^2$ dans les formules (I-2) et (I-3).

**4.** Polyimide selon la revendication 2, dans lequel, quand n n'est pas 0, au moins l'un des groupes $R^1$ et $R^2$ dans la chaîne principale de la formule (I) doit comprendre le groupe méthylène substitué.